# EUROPEAN PATENT APPLICATION

(11) **EP 3 048 168 A1**
(43) Date of publication of application: **27.07.2016**
(21) Application number: 15195977.2
(22) Date of filing: 24.07.2008
(51) Int. Cl.: C12N 5/071, C12Q 1/02

(54) **METHODS OF REDUCING INTRACELLULAR FATS FROM MAMMALIAN CELLS**

(30) Priority: 27.07.2007 US 935151 P
(62) Divisional of application: 08782329.0
(71) Applicant: Vesta Therapeutics, Inc., Durham, NC 27713 (US)
(72) Inventor: Ruiz, Joseph Charles, Durham, NC 27713 (US)
(74) Representative: Nederlandsch Octrooibureau

(57) **Abstract**

The present invention provides methods of reducing or clearing fat from mammalian cells. The method comprises culturing the cells in an environment that facilitates: 1) reduction of *de novo* fatty acid synthesis, 2) activation or synthesis of fatty acid oxidizing enzymes, and/or 3) export of lipids out of the cells. Cells substantially free of fatty acid are also provided in this invention.

## Description

### CROSS REFERENCE TO RELATED APPLICATIONS

The present application claims priority to US Provisional Application No. 60/935,151 filed July 27, 2007, the disclosure of which is incorporated herein by reference in its entirety.

### FIELD OF THE INVENTION

The present invention relates generally to methods to control some of the biological variations between tissue samples. More particularly, the present invention relates to methods of clearing or reducing fat from mammalian (e.g., liver) cells.

### BACKGROUND OF THE INVENTION

*In vitro* cell cultures, particularly cultures of mammalian cells, provide an invaluable resource to study not only the biological behavior of those cells, but also the effect of extrinsic compounds (*e**.**g.*, pharmaceuticals) on that behavior. While "immortalized" or "transformed" cells lines can be useful in this respect, it is well appreciated in the art that the very process of immortalization introduces genetic mutations, which are often unknown, that can compromise data obtained using them. To obviate this possibility, researches often seek "primary" cells for their studies (*i.e.,* cells that are freshly harvested from mammalian tissue).

The use of primary cells, however, poses a unique set of challenges. For example, tissue donors (particularly human donors) are perennially in short supply. Moreover, most cell types are not "renewable" and comparisons between tissues from genetically different animals need to account for those genetic differences, which is often not possible. In short, controlling for biological variations between donors of mammalian tissue remains difficult.

The pharmaceutical industry, wherein primary cells are sought for toxicology studies, provides a good example of this difficulty. Because the fat content of cells can adversely affect toxicity assays, researchers seek primary cells with the lowest fat content (*e.g*., less than about 30% intracellular fat). However, the incidence of obesity, which leads to an increase of intercellular free fatty acids and intrahepatic lipids, has dramatically risen over the past 25 years in the United States. Hence, a greater percentage of organs from Organ Procurement Organizations (OPOs) are steatotic (*i.e*., "fatty") and effectively useless. Because of the short supply of organ donors generally, and a shrinking percentage of donated organs with acceptable steatosis levels, a method of reducing or clearing intracellular fat is desirable and in need.

### SUMMARY OF THE INVENTION

In one embodiment, the present invention provides a method of reducing intracellular fatty acids from mammalian cells, including progenitors, *ex vivo* comprising: (a) obtaining a suspension of cells such as hepatic progenitor cells; and (b) culturing the cells in the presence of at least two of agent(s) that facilitate: 1) reduction of *de novo* fatty acid synthesis, 2) activation or synthesis of fatty acid oxidizing enzymes, and 3) activation of very low density lipoprotein (VLDL) production, for a period of time sufficient to reduce the total amount of intracellular fatty acids from the mammalian cells. While the mammalian tissue is preferably human tissue, and more preferably human liver, the inventive method may be applied to pancreatic, intestinal, cardiac and skeletal muscle. The cells may be fetal, neonatal or adult tissue, including cryopreserved cells and/or tissue.

Fatty acid biosynthesis may be inhibited by any of C75 related molecules (e.g., TOFA and C75), bile acids (cholic acid and chenoxycholic acid), activators of the FXR nuclear receptor, SHP, repressors of SREBP-1c or combinations thereof.

Fatty acid oxidation may be activated through transcription, translation, or activation of fatty acid oxidizing enzymes. For example, fatty acid oxidation may be achieved through transcriptional or translational activation of carnitine palmitoyltransferease (CPT-1), medium chain acyl-CoA-dehydrogenase (MCAD) and/or long chain acyl-CoA dehydrogenase (LCAD). Compounds suitable to activate fatty acid oxidation in this manner may be fibrates (e.g., bezafibrate, fenofibrate, clofibrate), PPAR family agonists, synthetic PPAR agonists (e.g., GW 501516 and GW0742), thiazolindinediones (e.g., pioglitozone and rosiglitazone), epoxyeicosatrienoic acids (e.g., 14,15 dihydroxyeicosatrienoic acid), CPT-1, MCAD, LCAD, or combinations thereof. Additionally, certain embodiments of the invention may contain an anti-oxidant is added to decrease intracellular oxidative damage. In another embodiment of the invention, the fatty acid oxidator is removed or suppressed after maintaining stable levels of steatosis.

Compounds suitable to activate VLDLs can include choline, choline derivates (e.g., choline, lysophosphatidylcholine, phosphatidylcholine, and, phosphatidylethanolamine), saturated fatty acids (e.g., lauric acid, palmitic acid, myristic acid, arachidic acid), monosaturated fatty acids (e.g., oleic acid, palmitoleic acid), polyunsaturated fatty acids (e.g., arachidonic acid, eicosapentaenoic acid), or combinations thereof. Hence, other compounds suitable to activate VLDL include agents that stimulate phosphatidylcholine synthesis and/or phosphatidylethanolamine N-methyltransferase (PEMT) activity.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE DRAWINGS

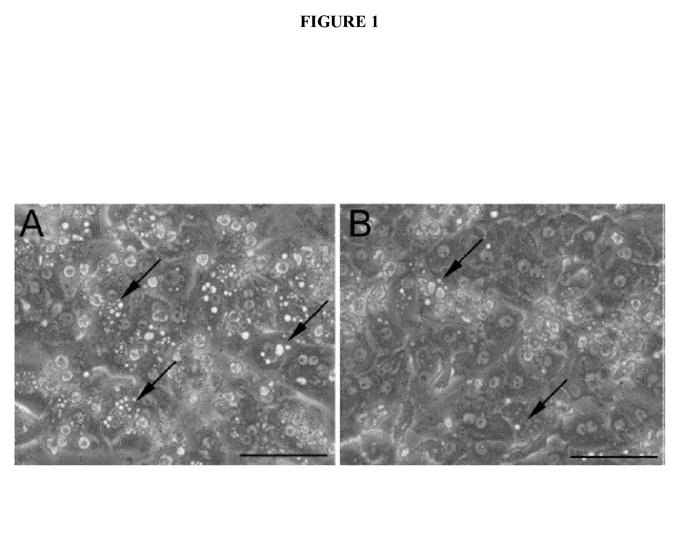

**Figure 1** is a phase contrast showing intracellular lipids deposits in hepatocytes before (panel **A**), and after (panel **B**) incubation with intracellular lipid clearing agents as described herein. Cryopreserved hepatocytes were plated in Williams E complete media, overlaid with Matrigel® on day 1, treated with the lipid clearing agents on day 2, and photographed on day 4. The arrows denote intracellular lipid deposits (white circular objects). The magnification bar represents 100 microns.

### DETAILED DESCRIPTION OF THE INVENTION

The present invention relates to a method of reducing or clearing fats from mammalian cells. While most, if not all, of the discussion and examples of the method will be with reference to human-derived hepatic cell populations, the teachings herein should not be limited to cells of the liver. In fact, one of ordinary skill in the art may be expected to apply the teachings herein to any mammalian cells in need of intracellular fat reduction (e.g., adipocytes, neurons, cardiomyocytes). Accordingly, the scope of the present invention is intended to mammalian cells of any and all tissues.

The broad applicability of the invention notwithstanding, cells of the liver are a preferred cell type for application of the instant invention at least because high levels of steatosis, or fatty liver degeneration, is present in between 13 and 50 percent of donor livers. In these cases, fat clearing or reducing methods may be particularly applicable and appropriate.

Unless explicitly stated otherwise, the term "reduce or reduction" is defined as "to become diminished" or "a lessening" (*e.g*., in the total amount or concentration of intracellular fat). While in some embodiments, the present invention may be used to "clear" (*i.e.,* substantially remove most intracellular fat), unless explicitly mentioned otherwise, the term "clear" is intend to mean a "reduction" of intracellular fat to a range of about 5-10% intracellular fat and not necessarily a complete "elimination" of fat. The term "total clearance" is intended to mean a "reduction" of intracellular fat to a range equal to or less than 5%, preferably less than about 1% intracellular fat, and can include the substantial elimination of all intracellular fat.

Cells "in need" of intracellular fat reduction or clearance are any population deemed to benefit from same. The present invention does not contemplate any particular concentration of intracellular fat to warrant the "need" for fat reduction. In the case of liver tissue, hepatic cells having greater than 30% fat are typically unusable for transplantation or toxicity assays. Hence, hepatic cells of greater than 40% or 50% fat would be in "need" of fat reduction for transplantation and/or toxicity assays. However, it may be desirable to reduce the fat content from cells (*e.g.,* hepatic cells) that have otherwise "acceptable" levels of fat. For example, hepatic cells have less than 30% fat may be nonetheless "in need" of fat reduction in order to meet or maintain experimental requirements.

In one embodiment of the invention, intracellular fatty acids are reduced by incubating cells in need of fat reduction in the presence of drugs that manipulate biological pathways that regulate fatty acid metabolism, catabolism, and/or export. Without being held to or bound by theory, the present inventors believe that steatosis is mediated by an imbalance between fatty acid uptake and *de novo* biosynthesis on one hand and oxidation and export on the other. Thus, steatosis occurs when uptake or biosynthesis exceeds the ability of the liver to oxidize and/or export the lipid. Hence, the present invention attempts to regulate steatosis, in part, through exogenous agents that either inhibit lipid biosynthesis, upregulate oxidative enzymes, or promote very low density lipoprotein (VLDL) secretion.

By following the teachings of the instant invention, an artisan can control for the range of intracellular fat in any given cell population. For example, if suspensions of liver cells A and B have an intracellular fat content of 60 units and 80 units, respectively, and a fat content of 20 units is desirable, following the inventive method, the artisan may be able to reduce the fat content in suspension A by about 67% and suspension B by 75% to arrive at a predetermined level (or range of levels) of intracellular fat content. In this way, one need not wait for an "ideal" donor to obtain liver cells of a desirable fat content, but may subject the liver of one or more donors to the inventive fat-reducing method to obtain populations of liver cells that have "control" levels of fat. In this way, the instant invention enables the production of cell populations standardized for intracellular fat. The cells have multiple applications, including toxicity assays, and in bio-assist devices and cell therapy.

The present invention enables the reduction of intracellular fat levels, preferably without interfering with normal cellular function. The present inventors have found that, with most agents, a single agent has little, if any, effect on reducing steatosis. However, when used in combination, these reagents surprisingly act in synergy to reduce fat levels. More specifically, the inventors were the first to propose that when inhibiting fatty acid synthesis in combination with activating fatty acid oxidation and/or secretion (i.e., perturbing two or more fatty acid pathways), there is a synergistic effect on reduction of steatosis.

The following examples are illustrative of the invention, but the invention is by no means limited to these specific examples. A person of ordinary skill in the art will find in these examples but one means to implement the instant invention. Further, while the instant examples have been present in the context of hepatocytes for experimental convenience, the methods and reagents described herein can be readily translated to other cell lines and cell types by one of ordinary skill in the art from the teachings disclosed below.

### Intracellular fat reduction via inhibition of lipid biosynthesis

De novo fatty acid biosynthesis is regulated, in part, by the LXR nuclear receptor. The LXR nuclear receptor can active numerous transcription factors (such as SREBP-1c), which in turn can activate a number of genes involved in lipogenesis. Therefore, in one embodiment of the present invention, cells in need of intracellular fat reduction are incubated in media with reagents (such as, but not limited to, cholic acid, chenodeoxycholic acid, oleic acid, TOFA, FAS, and/or MEDICA) that target proteins involved in de novo fatty acid biosynthesis. Typically a final concentration of 50 to 500 µM cholic acid, 50 to 200 µM chenodeoxycholic acid, 25 to 100 µM oleic acid, 1 to 10 µg/mL TOFA, 5 to 50 µg/mL FAS, or 2 to 70 µM MEDICA is sufficient to minimally affect fatty acid synthesis.

Recently, it was demonstrated that non-toxic levels of bile acids activates the FXR nuclear receptor, which in turn activates a repressor of the LXR nuclear receptor, called SHP. Therefore, in another embodiment of the present invention, cells in need of intracellular fat reduction are incubated in media with reagents such as, but not limited to, cholic and chenodeoxycholic acid. that target the repression of the LXR nuclear receptor.

### Intracellular fat reduction via activation fatty acid oxidation enzymes

Fatty acid oxidation is another pathway in which steatosis can be regulated. β-oxidation occurs in both mitochondria and peroxisomes. Mitochondria catalyze the β-oxidation of the bulk short-, medium-, and long-chain fatty acids derived from diet, and this pathway constitutes the major process by which fatty acids are oxidized to generate energy. Additionally, long-chain and very-long-chain fatty acids (VLCFAs) are also metabolized by the cytochrome P450 CYP4A ω-oxidation system to dicarboxylic acids that serve as substrates for peroxisomal β-oxidation. Thus, in one embodiment of the present invention, cells in need of intracellular fat reduction are incubated in media with peroxisome proliferator-activated receptor α (PPAR α) activators, which up-regulate enzymes involved in regulating fatty acid oxidation. Typically, 50 to 500 µM bezafibrate, 0.2 to 2 µM GW501516, 1 nM to 20 nM GW0742, 10 to 100 µM Fenofibrate, 100 to 500 µM Clofibrate, 10 to 100 WY-14643, 2 to 25 µM Rosiglitazone, 2 to 25 µM Pioglitazone, 2 to 25 µM 14,15-DHET, or 500 to 2000 ppm NO-1886 is sufficient to support fatty acid oxidation.

### Intracellular fat reduction via activation of VLDL secretion

Liver is the major organ for the synthesis and secretion of plasma lipoproteins in mammals. Triglycerides are but one type of fat that is involved in steatosis. Triglyerides are packaged as very low density lipoproteins (VLDL) for cellular export. Thus stimulation of intrahepatic lipid export is a target for fat reduction.

Triglycerides are packaged as VLDLs for cellular export. Additionally, VLDLs comprise about 60% phosphatidylcholine (PC), and without choline, VLDLs cannot be made, triglycerides cannot be exported, and in turn hepatocytes become steatotic. About 70% of the PC pool is synthesized from dietary choline. Moreover, choline deficient diets in animals and humans cause intrahepatic accumulation of triglycerides and hepatic steatosis in rats. Therefore, in one embodiment of the present invention, cells in need of intracellular fat reduction are incubated in media with additional choline to increase the production of hepatic PC and thus promote triglyceride export, and in turn reduce steatosis. In another embodiment of the present invention, other drugs such as lysophosphostidylcholine, phosphatidylcholine, phosphatidylethanolamine, lauric acid, palmitic acid, and myristic acid can be used to activate VLDL synthesis and/or secretion. Typically, a final concentration of 50 to 200 µM choline, 50 to 500 µM lysophosphatidylcholine, 50 to 500 µM phosphatidylcholine, 50 to 500 µM phosphatidylethanolamine, 100 to 1000 µM lauric acid, 100 to 1000 µM palmitic acid, or 100 to 1000 µM myristic acid is sufficient to support high VLDL synthesis and/or secretion.

The following table summarizes, in part, certain embodiments of the present invention. The concentration listed (in both Tables 1 and 2) is the concentration of the reagent in the media used to incubate cells in need of fat reduction.

**Table 1.**

| **Reagent Name** | **Min (µM)** | **Max (µM)** | **Preferred (µM, unless otherwise noted)** |
|---|---|---|---|
| **Inhibitors of FA synthesis** | | | |
| Cholic acid | 50 | 500 | 200 |
| Chenodeoxycholic acid | 50 | 200 | 100 |
| Oleic acid | 25 | 100 | 80 |
| 5-(tetradecyloxyl)-2-furancarboxylic acid, an inhibitor of acetyl-CoA carboxylase (TOFA) | 1µg/ml | 10µg/ml | 5µg/ml |
| C75, 4-methylene-2-octyl-5-oxo-tetrahydro-furan-3-carboxylic acid, an inhibitor of fatty acid synthase (FAS) | 5µg/ml | 50µg/ml | 20µg/ml |
| b,b,b',b'-tetramethylhexadecanoic acid, an inhibitor of acetyl-CoA carboxylase (MEDICA) | 2 | 70 | 50 |

| **Reagent Name** | **Min (µM)** | **Max (µM)** | **Preferred (µM,)** |
|---|---|---|---|
| **Activators of FA oxidation** | | | |
| Bezafibrate | 50 | 500 | 200 |
| GW501516 | 0.2 | 2 | 1 |
| GW0742 | 1 nM | 20 nM | 10 nM |
| Fenofibrate | 10 | 100 | 75 |
| Clofibrate | 100 | 500 | 300 |
| 4- chloro 6-(2,3 xylindine)-2 pyrmidinylthioacetic acid (WY-14643) | 10 | 100 | 75 |
| Rosiglitazone | 2 | 25 | 10 |
| Pioglitazone | 2 | 25 | 10 |
| 14, 15 dihydroxyeicosatrienoic acid (14,15-DHET) | 2 | 25 | 10 |
| NO-1886 [ibrolipim; 4-(4-bromo-2-cyano-phenylcarbamoyl)-benzyl]-phosphonic acid diethyl ester] | 500ppm (parts per million) | 2000ppm | 1000ppm |
| **Activators of VLDL synthesis and/or secretion** | | | |
| Choline | 50 | 200 | 100 |
| Lysophosphatidylcholine | 50 | 500 | 200 |
| Phosphatidylcholine | 50 | 500 | 200 |
| Phosphatidylethanolamine | 50 | 500 | 200 |
| Lauric acid (saturated fatty acid) | 100 | 1000 | 400 |
| Palmitic acid (saturated fatty acid) | 100 | 1000 | 400 |
| Myristic acid (saturated fatty acid) | 100 | 1000 | 400 |
| Arachidic acid (saturated fatty acid) | 100 | 1000 | 400 |
| Oleic acid (monosaturated fatty acid) | 100 | 1000 | 400 |
| Palmitoleic acid (monosaturated fatty acid) | 100 | 1000 | 400 |
| Arachidonic acid (polysaturated fatty acid) | 25 | 250 | 100 |
| Eicosapentaenoic acid (polysaturated fatty acid) | 25 | 250 | 100 |

While some embodiments of the present invention may comprise the use of one reagent, the present invention also contemplates the use of two or more reagents in combination. Preferably, when a combination of reagents is used, at least one reagent is selected from each of the following categories: inhibitors of lipid biosynthesis, activators of oxidative enzymes, or activators of VLDL secretion. In fact, without being held to or bound by theory, the present inventors believe that use of a reagent from a single category may be inefficient, if not ineffectual, in reducing intracellular lipids because a cell may compensate for the inhibition of one pathway, for example, by upregulating another. Hence, a combination of agents from two, preferably three, of categories aforementioned may be desirable. Table 2 provides some preferred "cocktail" combinations.

**Table 2.**

| **Reagent Name** | **Preparation No. (Concentration in µM,** * **=µg/mL)** | | | | | | | | | | | | | | | | | | | | | | | | |
|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
| Cholic acid | 200 | 200 | 200 | 200 | 200 | | | | | | | | | | | | | | | | | | | | |
| Chenodeoxycholic acid | | | | | | 100 | 100 | 100 | 100 | 100 | | | | | | | | | | | | | | | |
| Oleic acid | | | | | | | | | | | 80 | 80 | 80 | 80 | 80 | | | | | | | | | | |
| TOFA | | | | | | | | | | | | | | | | 5* | 5* | 5* | 5* | 5* | | | | | |
| MEDICA | | | | | | | | | | | | | | | | | | | | | 50 | 50 | 50 | 50 | 50 |
| Benzafibrate | 200 | | | | | 200 | | | | | 200 | | | | | 200 | | | | | 200 | | | | |
| GW501516 | | 1 | | | | | 1 | | | | | 1 | | | | | 1 | | | | | 1 | | | |
| Fenofibrate | | | 75 | | | | | 75 | | | | | 75 | | | | | 75 | | | | | 75 | | |
| WY-14643 | | | | 75 | | | | | 75 | | | | | 75 | | | | | 75 | | | | | 75 | |
| 14,15-DHET | | | | | 10 | | | | | 10 | | | | | 10 | | | | | 10 | | | | | 10 |
| Choline | 100 | | | | | 100 | | | | | 100 | | | | | 100 | | | | | 100 | | | | |
| Lysophosphatidylcholine | | 200 | | | | | 200 | | | | | 200 | | | | | 200 | | | | | 200 | | | |
| Arachidic acid | | | 400 | | | | | 400 | | | | | 400 | | | | | 400 | | | | | 400 | | |
| Palmitoleic acid | | | | 400 | | | | | 400 | | | | | 400 | | | | | 400 | | | | | 400 | |
| EPA | | | | | 10 | | | | | 10 | | | | | 10 | | | | | 10 | | | | | 10 |

The following examples are illustrative of the invention, but the invention is by no means limited to these specific examples. A person of ordinary skill in the art will find in these examples but one means to implement the instant invention.

**Hepatocytes show reduced intrahepatic lipids and improved cell morphology after treatment with lipid clearing agents:** Cryopreserved steatotic hepatocytes from a donor were plated and propagated in Williams E media supplemented with 200 µM cholic acid, 200 µM bezafibrate, and 100 µM choline for two days. Before plating, nearly all cells contained multiple intracellular lipid deposits **(****Figure 1****).** Upon 48 hours of treatment in the "cocktail" of agents, however, there was a noticeable decrease in lipid deposits, by about 80%. Indeed, the majority of the treated cells lacked the larger intracellular lipid deposits found in untreated cells **(****Figure 1****).**

The present inventors have also discovered that the quality of the fat-reduced hepatocytes is also improved by the inventive method. More specifically, fat-reduced hepatocytes exhibited morphology comprising cord-like structures interspersed with clear channels, the presumptive biliary canaliculi, which is indicative of hepatoblasts *in vivo.* Surprisingly, this data demonstrate that the inventive fat-reducing methods do not appear to adversely affect cell function, but rather assist and improve that function, as compared to steatotic hepatocytes.

**Visualizing intracellular steatosis:** To visualize intracellular lipid deposits, cells were stained with Oil-Red O (Solvent Red 27, Sudan Red 5B, C.I. 26125, C₂₆H₂₄N₄O), a lysochrome (e.g., fat-soluble) diazo dye for staining of neutral triglycerides and lipids and some lipoproteins. Briefly, _cells are fixed in 10 % formalin, rinsed 3X with PBS, stained with Oil Red O for 15 minutes, and washed 3X with water before photographing the cells. Nile Red is another staining agent that can be used to visualize intracellular lipid deposits in a similar manner.

**Quantifying intracellular steatosis:** Isopranol can be used to elute the Oil Red O stain, if any, from the cells; the absorbance (A₅₄₀) of the elutant can be used to compare the level of Oil Red O staining (i.e., level of intrahepatic lipid droplets) of treated cells to untreated cells. Another approach is to take electronic photomicrographs of the cells and analyzing them (*e.g*., with Metamorph™ software) to calculate the percent area that is taken by lipid deposits in a microscopic field. The percent *area* steatosis can then be converted into percent *volume.* As a control, hepatocytes derived from pediatric donors may be used, which cells are typically non-steatotic. A direct measure of steatosis levels is to determine the amount of triglycerides (TG) in the hepatocyte cultures, Because TG is the form in which intrahepatic lipids are stored, determining TG levels in cell lysates can provide a quantitative measurement of intrahepatic lipid levels.

The inventive method enables the generation of cell populations from diverse donors to be standardized for intracellular fat content. These fat-reduced cells can be used for a variety of proposed studies, and expand the range of non-transplantable livers for academic, clinical and pharmaceutical research. In yet other embodiments, the inventive method enables the clearance of intracellular fat to a level that is not present or known in the art. For example, the present invention provides a population of hepatic cells with total clearance of fat (less than about 5%, preferably less than about 3%, more preferably less than about 1%, and most preferably essentially free of intracellular fat). The term "about" has been recited here and throughout the specification to account for variations, which can arise from inaccuracies in measurement inherent and understood by those of ordinary skill in the chemical and pharmaceutical arts.

While the invention has been described in connection with specific embodiments thereof, it will be understood that it is capable of further modifications and this application is intended to cover any variations, uses, or alterations of the invention following. In general, the principles of the invention and including such departures from the present disclosure as come within known or customary practice within the art to which the invention pertains and as may be applied to the essential features hereinbefore set forth and as follows in the scope of the appended claims.

## Claims

1. A method of reducing intracellular fatty acids from mammalian cells comprising:
(a) obtaining mammalian cells in need of fatty acid clearing; and
(b) incubating the mammalian cells with one agent from at least two of the following groups:
(i) inhibitor of fatty acid biosynthesis
(ii) activator of fatty acid oxidizing enzymes; and
(iii) activator of very low density lipoprotein (VLDL) production,
for a period of time sufficient to reduce the total amount of intracellular fatty acids from the mammalian cells.

2. A method of reducing intracellular fat from a population of cells to a predetermined level comprising (a) obtaining mammalian cells and (b) incubating the mammalian cells with one agent from at least two of the following groups:
(i) an inhibitor of fatty acid biosynthesis;
(ii) an activator of fatty acid oxidizing enzymes;
(iii) an activator of very low density lipoprotein (VLDL) production,
for a period of time sufficient to reduce the total amount of intracellular fatty acids to the predetermined level of intracellular fat in the suspension of mammalian cells.

3. The method of claim 1 or 2 in which the mammalian cells are human cells or adult liver cells.

4. The method of claim 3 in which the cells are hepatocytes or adipocytes, preferably primary hepatocytes.

5. The method of claim 1 or 2 in which the inhibitor of fatty acid biosynthesis is cholic acid, chenodeoxycholic acid, oleic acid, C75, TOFA, FAS, or MEDICA, preferably cholic acid.

6. The method of claim 5 in which the cholic acid is present at a concentration between 0 and 500 µM, preferably between 150 and 250 µM.

7. The method of claim 1 or 2 in which the activator of fatty acid oxidizing enzymes is a fibrate, a PPAR agonist, preferably bezafibrate, GW501516, GW0742, or combinations thereof; a thiazolindinedione; an epoxyeicosatrienoic acid: CPT-1; MCAD; LCAD; or a combination thereof

8. The method of claim 7 in which bezafibrate is present at a concentration between 150 µM and 250 µM.

9. The method of claim 7 in which GW0742 is present at a concentration between 0 and 20 µM.

10. The method of claim 7 further comprising an anti-oxidant.

11. The method of claim 1 or 2 in which the activator of VLDL production is choline, a choline derivate, a saturated fatty acid, a monosaturated fatty acid, a polyunsaturated fatty acids, or a combination thereof, preferably choline.

12. The method of claim 11 in which the choline is present at a concentration between 0 and 200 µM.

13. The method of claim 1 or 2 in which fatty acids are cleared by incubating with cholic acid, bezafibrate, and/or choline.

14. The method of claim 1 or 2 in which the suspension is incubated with 0 to 200µM cholic acid, 0 to 10 nM GW0742, and 0 to 70 µM choline.

15. The method of claim 1 or 2, in which the suspension is incubated with 200 µM cholic acid, 200 µM bezafibrate, and 100 µM choline.

16. A mature hepatocyte essentially free of fatty acids.

17. A population of cells obtainable by the method of claim 1 or 2.
